# EUROPEAN PATENT SPECIFICATION

(11) **EP 3 006 570 B1**
(45) Date of publication and mention of the grant of the patent: **07.06.2023**
(21) Application number: 14738868.0
(22) Date of filing: 27.05.2014
(51) Int. Cl.: C12Q 1/6883

(54) **METHOD FOR DETERMINING THE RISK OF DEVELOPING AN INFECTIOUS DISEASE**
VERFAHREN ZUR BESTIMMUNG DES RISIKOS EINE INFEKTIÖSE KRANKHEIT ZU ENTWICKELN
MÉTHODE DE DÉTERMINATION DU RISQUE DE DÉVELOPPER UNE MALADIE INFECTIEUSE

(30) Priority: 27.05.2013 ES 201330768
(43) Date of publication of application: 13.04.2016
(73) Proprietor: Fundación para la Investigación Biomédica del Hospital Universitario de la Paz, 28046 Madrid (ES); Llamas Matías, Miguel Ángel, 28011 Madrid (ES); Empireo, S. L., 28004 Madrid (ES)
(72) Inventor: LLAMAS MATÍAS, Miguel Ángel, E-28011 Madrid (ES); LÓPEZ COLLAZO, Eduardo, E-28046 Madrid (ES)
(74) Representative: Pons
(86) International application number: PCT/ES2014/070434
(87) International publication number: WO 2014/191601

(56) References cited:
- WO-A1-2004/005539
- WO-A2-2012/075506
- CHIA-TE KUNG ET AL: "Plasma nuclear and mitochondrial DNA levels as predictors of outcome in severe sepsis patients in the emergency room", JOURNAL OF TRANSLATIONAL MEDICINE, BIOMED CENTRAL, LONDON, GB, vol. 10, no. 1, 21 June 2012 (2012-06-21), page 130, XP021116132, ISSN: 1479-5876, DOI: 10.1186/1479-5876-10-130
- SURSAL TOLGA ET AL: "Plasma bacterial and mitochondrial DNA distinguish bacterial sepsis from sterile systemic inflammatory response syndrome and quantify inflammatory tissue injury in nonhuman primates", SHOCK,, vol. 39, no. 1, 1 January 2013 (2013-01-01), pages 55-62, XP009171328, ISSN: 1540-0514, DOI: 10.1097/SHK.0B013E318276F4CA
- ANDREAS MEISEL ET AL: "Predicting Post-Stroke Infections and Outcome with Blood-Based Immune and Stress Markers", CEREBROVASCULAR DISEASES, vol. 33, no. 6, 1 January 2012 (2012-01-01), pages 580-588, XP055147833, ISSN: 1015-9770, DOI: 10.1159/000338080
- QIN ZHANG ET AL: "Circulating mitochondrial DAMPs cause inflammatory responses to injury", NATURE, NATURE PUBLISHING GROUP, UNITED KINGDOM, vol. 464, no. 7285, 4 March 2010 (2010-03-04), pages 104-107, XP002659275, ISSN: 0028-0836, DOI: 10.1038/NATURE08780

## Description

The present invention is an *in vitro* method that determines the risk of developing an infectious disease. This includes the detection and /or quantification of a mitochondrial compound in a biological sample. It also includes the use of a kit for the same purpose. Therefore, this invention could be included in the field of biomedicine.

### BACKGROUND OF THE INVENTION

Human sepsis is a systemic infection disease triggered by the systemic inflammatory response syndrome (SIRS). It is the only one among the first 10 major diseases in the developed countries that hasn't changed its mortality rate in over 30 years (Artero A. et al, 2010 Journal of Critical Care 25: 276-281).

This data can be explained by two causes; first, the progress of science in the past three decades has allowed for the modification of mortality rates mainly due to the improvement of antibiotic treatments; the second, is due to early treatment guidelines, which has also, albeit moderately, decreased mortality rate. However, all this progress hasn't stopped a 4.2% yearly increase that affects 1.7 million septic patients in 2009 in the US alone.

According to this data and its present population of 314 million inhabitants, US has a prevalence of septic patients (sepsis as primary diagnosis and secondary to another disease) of approximately 0.67%, at a cost of approx.16 trillion dollars in aprox. 1.7 million patients, which is an expense of over 9000$ per patient.

A sepsis patient can present a variety of etiologies, s/he may develop a primary infection or it may be secondary to hundreds of other possible diseases. A sepsis patient may come from a coronary, trauma, ER or any other hospital department; s/he may have no prior medical record or may have a heart condition, suffer from diabetes or have a very complex medical record or quite the opposite; have a straightforward medical record. In other words, sepsis patients are a very difficult population to study and standardize, nevertheless, they all share a common problem; their immune system has been dysregulated because of a massive infection attack.

Antibiotic treatments have improved, but the microorganisms involved the infection have also evolved and nowadays there are increasing populations that turn out to be multi-resistant to antibiotic treatment. Among these bacteria causing septicemia we find the gram-negative bacteria for example, the *Pseudomonas* genus and *Escherichia coli* species. Yet gram-positive bacteria is also associated with septicemia, for example, the bacteria genus *Streptococcus, Pneumococcus, Staphylococcus,* more specifically within the latter, the S. *aureus* species, both methicillin-resistant and methicillin non-resistant strains. Furthermore sepsis has also been reported as being caused not only by bacteria (bacteremia) but also by viruses (viremia) or by fungi (fungemia) (Phillip Dellinger R. et al. 2013 Crit Care Med 41 :580-637).

Some septicemia diagnostic methods have already been developed like, for example Septifast^{®}, a diagnostic PCR kit that identifies 16 and 6 of the main bacteria and fungi that respectively cause human septicemia. Sepsitest^{®}, identifies 54 bacteria and 5 fungi; VYOO^{®}, identifies 33 anaerobic bacteria and 6 fungi; or Plex-ID^{®}, identifies a wide sample of bacteria, viruses, fungi and certain parasites, as well as offer information about their resistance, virulence and type of strain. Nevertheless, in spite of progress made in septicemia detection, no significant decrease in mortality nor in people suffering from septicemia has been recorded. There are treatments to make attempts at palliating and controlling the effects of sepsis, for example, maintaining levels of glucose or blood pressure, diminishing coagulation, blocking bacterial lipopolysaccharide (LPS) receptors or controlling the levels of some cytokines secreted into blood stream (Phillip Dellinger R. et al. 2013 Crit Care Med 41 :580-637; Sweeney DA, et al. 2008 Intensive Care Med 34: 1955-1960). However, there is no specific medication or strategies against sepsis and there is also an infinite number of causal agents that make an early diagnosis difficult.

Thus, due to all the motives exposed above, there is a need to find a method that allows to predict which patients are potential candidates to infectious disease and therefore, enable us also prevent septicemia. In this way patients that will be or have just been through surgery and that have a high risk of infection could be given an early antibiotic treatment whereas an indiscriminate use of antibiotics could be avoided in patients that are not at risk of developing an infectious disease .

### DESCRIPTION OF THE INVENTION

The scope of the invention is defined by the appended claims.

The technical problem this invention solves consists in providing an *in vitro* method to predict the risk of developing an infectious disease thus enabling a personalized and/or early treatment of the patient. This method allows to predict which patients will suffer from an infectious disease and therefore, also to prevent septicemia.

The invention's method allows us to know whether the patient will need an antibiotic treatment to prevent an infection. Therefore, this invention is also useful in determining which doses of antibiotics would be necessary in case they would be needed.

The method of the invention is especially useful in patients that will go into or that have already had surgery. In the first of these cases if the patient has an infection tolerance, an operation could be postponed or a preventive antibiotic therapy could be given and thus, the risk of developing an disease associated with tolerance to infections will be prevented.

In this present disclosure but not as part of the invention we prove that the detection of mitochondria compounds in a biological sample of person is associated to a state of tolerance to infections and that the use of mitochondria compounds is useful to determine the existence of a risk of developing an infection in patients that haven't had prior infections and that haven't previously had a transplant. These subjects not having received immunosuppressant treatment will forthwith be referred to as "the invention's patients".

In the present disclosure but not as part of the invention the usefulness of this method is shown in patients that have had acute myocardial infarction (AMI) and also proves its usefulness for patients that will go through or have gone through major surgery and did not have an infection at the time of the operation. The usefulness of the method has also been proved in patients that have suffered a cerebral infarction.

In the present disclosure but not as part of the invention it is proven that patients that go through a process that releases mitochondria content into their blood are at higher risk of having an infectious disease. The invention's method allows for the prediction and therefore, the prevention of a bacterial infection after an injury or disease or after o before major surgery by determining the risk of developing an infection.

The invention's method is non-invasive, fast, simple and effective.

In the present invention "tolerance to infections" and also " being in a refractory state" refers to a state of innate immune system wherein it ignores, or does not detect and/or react against an infection caused by a bacteria, fungus, virus or parasite.

The infectious disease mentioned in the present invention refers to an infectious disease caused by bacteria, this includes gram-positive bacteria as well as gram-negative bacteria; a fungus, like for example *Candida albicans;* a virus, like for example an *Herpes virus* or *Haemofilus influenza;* or a parasite, like for example the one that causes malaria. In a preferred embodiment the infectious disease is caused by bacteria.

In the present invention "bacterial infection" refers to infections caused by either gram-positive or negative bacteria. In the present invention bacterial infection can be caused by for example gram-negative bacteria such as *Pseudomonas,* or *Escherichia coli* or *Serratia, or by* gram-positive bacteria *such* as *Streptococcus, Pneumococcus, Staphylococcus,* more specifically within the latter t*he S*. *aureus* species. Infections can be caused by methicillin-resistant or methicillin-non resistant strains. Infection can be caused by aerobic or anaerobic bacteria. Infections caused by anaerobic bacteria of the following genus *Actinomyces, Propioni bacterium, Eggertella, Eubacterium y Clostridium* are included.

The term "gram-positive" refers to bacteria that are stained dark blue or purple in accordance to gram's stains which are well-known by experts in the art. This characteristic is linked to the structure of the cells envelope. The cell envelope of gram-positive bacteria comprises the cytoplasmic membrane and a cell wall consisting of a peptidoglycan layer surrounding the former one that makes the cell wall retain the stain coloring while using the gram stain. Gram-negative bacteria have a second lipid membrane external to the cell wall and this cell wall is made of an even finer peptidoglycan layer than those of the gram-positive bacteria. Therefore, gram-negatives are bacteria that have a second lipid membrane external to the cell wall and its cell wall is not as thick as the walls of gram-positive bacteria and are not stained in dark blue or purple in Gram's stains.

The present disclosure describes how the state of tolerance to infections can be predicted by the detection of mitochondria compounds in the blood sample of a patient, thus allowing for the treatment to be adapted to each patient according to his /her immunological sate.

Therefore, a first aspect of the disclosure but not part of the invention refers to a method of obtaining useful data to predict the risk of developing an infectious disease that includes the detection and /or quantification of a mitochondria compound within a biological sample isolated from a subject. Henceforth we shall refer to this as "the first method of the disclosure but not part of the invention".

In this invention "predict the risk" means to determine the risk of developing an infectious disease before it has occurred.

"Mitochondrial compound" refers to a portion or structure that belongs to the intracellular organelle known by experts in the field as mitochondria, for example, nucleic acids (deoxyribonucleic acid, DNA), ribonucleic acid, RNA), proteins, membranes, glucids, lipids, and combinations thereof. Mitochondrial compound is found in the biological sample after the cell's rupture of any of the subject's cells. A mitochondria compound can be detected and /or quantified by molecular biology techniques known by experts in the field, like for example *Western blot, Southern blot, Northern blot,* real-time polymerase chain reaction (RT-PCR), PCR, chromatography, immunoassay (for example ELISA),enzyme assay and spectroscopy.

The term "biological sample" includes but without limitation, tissues and /or biological fluids of an individual, obtained via any methods known by experts in the field for being used to that end. The biological sample includes mitochondrial DNA, mitochondrial RNA and /or mitochondrial proteins.

The term "subject" refers to an individual, preferably a human being, who does not suffer from infection and that has not received any immunosuppressed treatment nor has had a transplant. For this reason, a preferred embodiment of the first aspect of the invention refers to a method in which the subject is human.

Another preferred embodiment of the first aspect of the disclosure but not part of the invention refers to the method in which the infectious disease is sepsis.

Another preferred embodiment of the first aspect of the disclosure but not part of the invention refers to the method, in which the subject has had or has acute myocardial infarction (AMI).

In the present invention "acute myocardial infarction" (AMI) refers to the pathology result of insufficient blood flow with tissue damage somewhere in the heart, generally caused by a coronary artery obstruction, frequently by the rupture of a theromatic plaque. AMI are sub classified into STEMI or NSTEMI, depending on whether the ST segment is elevated or not, respectively. ST segment is a region of the curve represented in an electrocardiogram which is recognized as such by the field expert. The classification "with or without ST elevation" refers to a characteristic that appears in electrocardiograms and which is recognized by the field expert; if the segment ST is elevated, it's a STEMI, if it is not elevated, it is an NSTEMI. These clinical traits called STEMI and NSTEMI define the presence or not of necrosis in the heart's tissue, respectively.

Another preferred embodiment of the first aspect of the disclosure but not part of the invention refers to the method wherein the subject has suffered a cerebral infarction.

Cerebral infarction (brain ischemia or brain stroke) refers to the cerebral vascular insult (or disease) caused by an ischemia process in which part of the brain tissue ceases to function due to a disturbance of blood supply. Ischemia is caused by the occlusion of the brain's arteries due to a thrombotic stroke or embolism. A cerebral infarction can be followed by a reperfusion which is known as a transient ischemic attack (TIA), but in most cases it causes permanent damage within ranging scales of severity from slight memory loss/ problems to extreme disability.

An even more preferred embodiment of the first aspect of the disclosure but not part of the invention refers to the method in which the subject will go through or has gone through surgery, preferably a major surgery.

In the present invention "surgery" refers to the mechanical handling of anatomic structures for a medical purpose, be it diagnosis, treatment or prognosis. "Major surgery" refers to for example, but without limitation, surgery of the digestive tract, surgery of the endocrine system, surgery of the nervous system, surgery of the circulatory system or surgery to repair damage from accidents.

Another even more preferred embodiment of the first aspect of the disclosure but not part of the invention refers to the method wherein the mitochondrial compound is selected from a list consisting of mitochondrial deoxyribonucleic acid (mtDNA), mitochondrial ribonucleic acid (mtRNA) and mitochondrial protein. More preferably, the mitochondrial compound is mtDNA. In a more preferred embodiment, mtDNA is the gene 12S rRNA.

Gene 12S rRNA (*Gene ID:* 6183, "mitochondrial ribosomal protein S12", *MRPS12,* of *Homo sapiens*), (SEQ ID NO: 1) has a low frequency of mutations, does not possess isoforms, and is a region that is rich in CpGs islands.

According to what is described here, another preferred embodiment of the first aspect of the disclosure but not part of the invention refers to a method in which the mitochondrial compound is a region rich in CpGs islands.

"CpGs islands" in the present invention refers to DNA regions in which the proportion of dinucleotides CG (Cytosine-Guanine) is over 40%.

Another even more preferred embodiment of the first aspect of the disclosure but not part of the invention refers to a method in which the biological sample is blood plasma, blood serum or blood.

In the method of the first aspect of the disclosure but not part of the invention the detection and/or quantification of the concentration of the mitochondria compound is done via one of the techniques selected from the list consisting of Western blot, PCR, chromatography, immunoassays, enzyme assay and spectroscopy. In a preferred embodiment, the technique used to determine the concentration of the mitochondria compound is PCR. Preferably, the PCR used is a quantitative PCR.

Another even more preferred embodiment refers to the method of first aspect of the disclosure but not part of the invention in which the quantitative PCR is carried out through the use of primers whose sequences comprise SEQ ID NO: 2 (sense primer, of sequence 5'-ccacgggaaacagcagtgatt-3') and SEQ ID NO: 3 (antisense primer, of sequence 5'-ctattgacttgggttaatcgtgtg-3') and the probe comprises SEQ ID NO: 4 (sequence 5'-tgccagccaccggg-3'). In other words the pair of primers used is the pair of two primers that comprises SEQ ID NO: 2 and SEQ ID NO: 3. Another even more preferred embodiment refers to the method wherein quantitative PCR is carried out through the use of the primers whose sequences consist of SEQ ID NO: 2 and SEQ ID NO: 3 and a probe consisting of SEQ ID NO: 4. That is to say, the pair of primers used is the pair of primers of sequence SEQ ID NO: 2 and SEQ ID NO: 3. The probe can be labeled with fluorophores for example, with fluorescein isothiocyanate, phycoerythrin, ethidium bromide or "SYBR^{®} Green".

A second aspect of the disclosure refers to an *in vitro* method used to predict the risk of developing an infectious disease that comprises detecting and /or quantifying a mitochondrial compound in a biological sample of a subject.

Further on we shall refer to this as the "second method of the disclosure

A preferred embodiment of the second aspect of the disclosure refers to the method wherein in addition the value of the concentration of the obtained mitochondrial compound is compared to the value of the concentration of the same mitochondrial compound from at least one control sample in order to find a significant difference.

Another even more preferred embodiment of the second aspect of the disclosure refers to the method that also comprises associating the significant difference with the risk of developing an infectious disease.

The term *"in vitro"* means that the method of the invention takes place outside the subject's body.

The term "control samples" or "reference sample" or "healthy volunteer sample", in the present invention refers to, for example, but without limitation, to the samples obtained from people who have no predisposition to develop infections, that have had no infection in a period of 1 to 20 days prior to sample extraction, that have no immunosuppressant diseases (for example, acquired immunodeficiency syndrome, AIDS; or lupus), that are not under immunosuppresant treatment (for example, transplant or future transplant patients) and that have no history of myocardial infarcts.

Data obtained in the detection and/or quantification of the mitochondrial compound, for example, mtDNA, from the control sample can be obtained at the same time, in other words, simultaneously to the obtaining of mitochondrial compound, for example mtDNA, data of the person who is being studied, or can be a reference value that has been previously established.

In the second method of the invention it considers a difference to be significant when the value of the sample under observation is at least 2 times higher than the average +/- standard deviation of the control sample or healthy volunteer sample.

In another preferred embodiment of the second aspect of the disclosure but not of the invention the reference sample and the subject's samples have been previously normalized prior comparison. "Normalization" refers to the deletion of experimental variations between the different samples.

A preferred embodiment of the second aspect of the disclosure refers to the method wherein the infectious disease is sepsis

Another preferred embodiment of the second aspect of the disclosure refers to the method wherein the subject has had an acute myocardial infarction.

In the case of the method in which the subject has had an acute myocardial infarction, the control sample can be the sample of an individual who has not had that disease.

Another preferred embodiment of the second aspect of the disclosure refers to the method wherein the subject has had a cerebral infarction.

In the case of the method wherein the subject has had a cerebral infarction, the control sample can be the sample of an individual who has not suffered from that disease.

An even more preferred embodiment of the second aspect of the disclosure refers to the method wherein the subject will go through or has gone through surgery, preferably major surgery.

Another further preferred embodiment of the second aspect of the disclosure but not part of the invention refers to the method wherein the mitochondrial compound is selected from a list consisting of mitochondrial RNA and mitochondrial protein. More preferably, the mitochondrial compound is mitochondrial DNA. In an even more preferred embodiment, the mitochondrial DNA is the gene 12S rRNA. Another preferred embodiment of the second aspect of the invention refers to the method wherein the mitochondrial compound is a region rich in CpGs islands.

In order to detect and /or quantify the mitochondrial compound of the first and second methods of the disclosure but not part of the invention the isolated biological sample can be treated either physically or mechanically in order to tear the tissue or the cell structures and thus release the intra cellular components into an aqueous or organic solution and thus prepare the nucleic acids or proteins for further analysis. Nucleic acids or proteins are extracted from the sample through procedures known by the experts in the field or that are commercially available.

Another further preferred embodiment of the second aspect of the disclosure refers to the method wherein the biological sample is blood plasma, blood serum or blood.

The concentration of the mitochondrial compound can be measured by copies of mtDNA/µl plasma.

In the method of the second aspect of the disclosure but not part of the invention the detection and /or determination of the concentration (quantification) of the mitochondrial compound is done through one of the techniques selected from the list consisting of Western Blot, real time-polymerase chain reaction (RT-PCR), PCR, chromatography, immunoassay, enzyme assay and spectroscopy. In a preferred embodiment, the technique used to determine mitochondrial compound's concentration is PCR. Preferably, the PCR used is Quantitative PCR.

On another hand, the detection and /or quantification can also be done through Real Time-PCR, so another preferred embodiment of the second and first aspects of the invention refers to a method wherein the detection and /or quantification of mitochondrial DNA (mtDNA) is carried out via RT-PCR. This RT-PCR process means the amplification of mitochondrial DNA extracted from the plasma sample. For every sample and every transcript of the analyzed genes a PCR reaction will be carried out individually. This process means the cyclical repetition of 3 phases: a DNA denaturing phase, a phase in which the oligonucleotide of the gene under study binds specifically to the strand of the denatured DNA, and an elongation phase starting from the joined oligonucleotide by means of which a new DNA strand will be synthesized. As it is a process measured in real time, it is necessary to use a fluorescent molecule to monitorize what happens along the whole process.

The quantification, on the other hand, can also be done by determining the level of mitochondrial protein. This protein quantification can be done with any known method used for this purpose by an expert of the area, like for example, but not only, immunodetection methods (such as Western blot, ELISA, immunohistochemistry, immunocytochemistry, immunofluorescence), methods based on isobaric labeling (such as iTRAQ Isobaric tags for relative and absolute quantitation, or ICAT -Isotope-Coded Affinity Tag) or isotopic labeling (such as SILAC -Stable Isotopes Labeling by Amino Acids in Cell Culture) or based on fluorescent labeling (such as 2D-DIGE - Difference in Gel Electrophoresis), as well as methods based on mass spectrometry (MRM, -Multiple Reaction Monitoring). This can also be done via probes fixed on a support.

An even more preferred embodiment refers to the method of the second aspect of the disclosure wherein the Quantitative PCR is carried out by using primers comprising SEQ ID NO: 2 and SEQ ID NO: 3 and with a probe comprising SEQ ID NO: 4. In other words, the pair of primers used is the pair of two primers comprising SEQ ID NO: 2 and SEQ ID NO: 3. Preferably, the primers are the pair of primers of the SEQ ID NO: 2 and SEQ ID NO: 3 sequences and the probe is the probe of SEQ ID NO: 4 sequence. The probe can be labeled with fluorophores, like for example, fluorescein isothiocyanate, phycoerythrin, ethidium bromide or "SYBR^{®} Green".

A third aspect of the disclosure but not part of the invention refers to the use of a kit comprising primers, probes and/or antibodies to carry out the first or second method of the disclosure

A preferred embodiment of the third aspect of the disclosure but not part of the invention refers to the use of a kit comprising primers and probes. Another even more preferred embodiment refers to the use of a kit that comprises primers and probe, wherein the primers comprise sequences SEQ ID NO: 2 and SEQ ID NO: 3 and the probe comprises sequence SEQ ID NO: 4, in order to predict the risk of developing an infectious disease.

An even more preferred embodiment refers to the use of a kit that comprises primers and probe, wherein primers are the pair of primers comprising sequences SEQ ID NO: 2 and SEQ ID NO: 3 and the probe comprises sequence SEQ ID NO: 4, more preferably the primers are sequences SEQ ID NO: 2 and SEQ ID NO: 3 and the probe is sequence SEQ ID NO: 4, in order to predict the risk of developing an infectious disease.

An even more preferred embodiment of the third aspect of the disclosure but not part of the invention refers to the use of the kit wherein the infectious disease is sepsis.

The kit referred to in the third aspect of the disclosure but not part of the invention can comprise at least one DNA polymerase or a fluorophore. Also the kit can comprise a mixture of deoxynucleotides triphosphate (dNTPs), a mixture of nucleotides triphosphate (NTPs), deoxyribonuclease (DNase), dithiothreitol (DTT), inorganic pyrophosphatase (PPI) and the necessary buffers for the enzymes provided in the kit.

Furthermore in the kit used in the third aspect of the disclosure but not part of the invention the probes, primers or antibodies can be placed on a solid support, for example, but not solely, crystal, plastic, tubes, plaques, multi-well plates , membranes, or any other support known by a specialist in the subject.

The present disclosure but not part of the invention also refers to a kit that comprises the necessary elements required to detect and /or quantify the presence of DNA mitochondrial rich in CpGs islands. The use of this kit could be used to predict the risk of developing an infectious disease, preferably sepsis. This kit can comprise primers and/or probes to detect mitochondria DNA rich in CpGs islands in a subject's biological sample. Also, the kit can also comprise some of the elements of the kit mentioned in the third aspect of the invention and also some of its elements can be placed on a solid support.

All through this patent description and claims the word "comprise" and its variations do not exclude other technical features, additives, components or steps. For experts in the subject, other objects, advantages and features of the invention will be derived in part from the description and in part also from the use of the invention. The following examples and figures are presented to illustrate and have no intention of being constrained to the present invention.

### BRIEF DESCRIPTION OF FIGURES

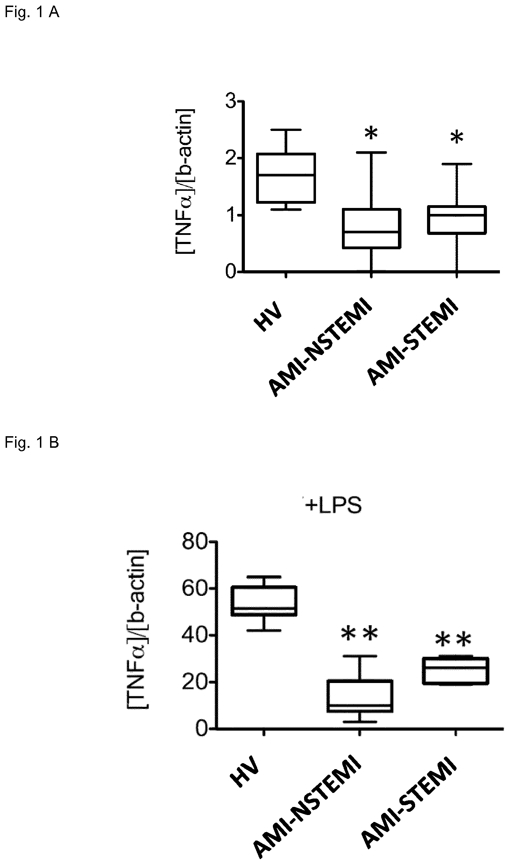

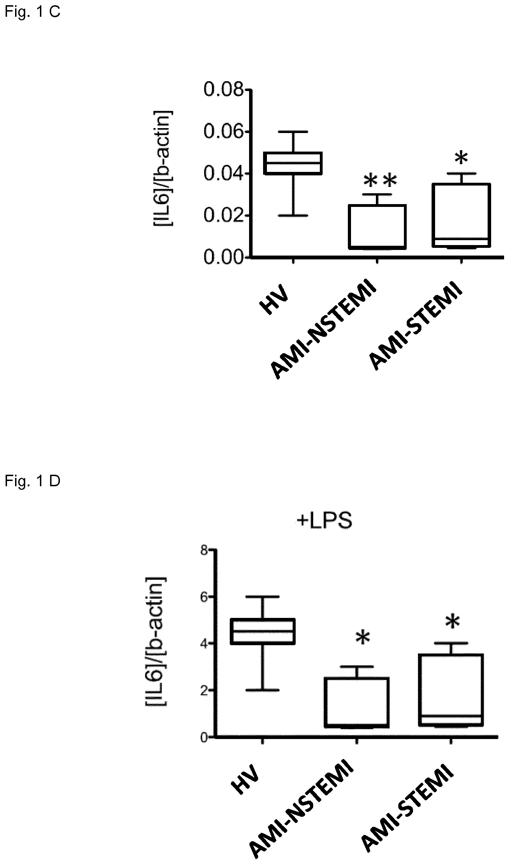

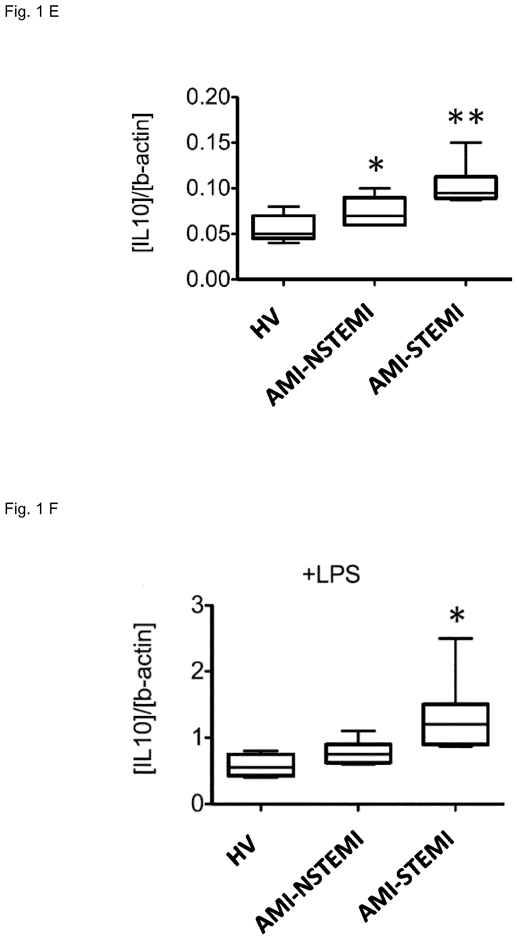

Fig.1. Represents the transformation/polarization towards a tolerant phenotype (also known as M2) of circulating macrophages/monocytes in Acute Myocardial Infarction (AMI) patients. The macrophages/monocytes isolated from AMI patients were treated (B,D and F) or not (A, C, and E) with LP3 (3h, 10ng/ml). Afterwards, the total messenger RNA (mRNA) was isolated and the cDNA (complementary DNA) was synthesized. Afterwards a quantification of TNFα (A, B), IL6 (C, D) and IL10 (E, F) levels was made. Values of healthy volunteers (HS), n=20; patients with: NSTEMI n= 24, STEMI n= 21. *p<0.05, **p<0.01 patients vs HV (ANOVA/Dunn) were represented.

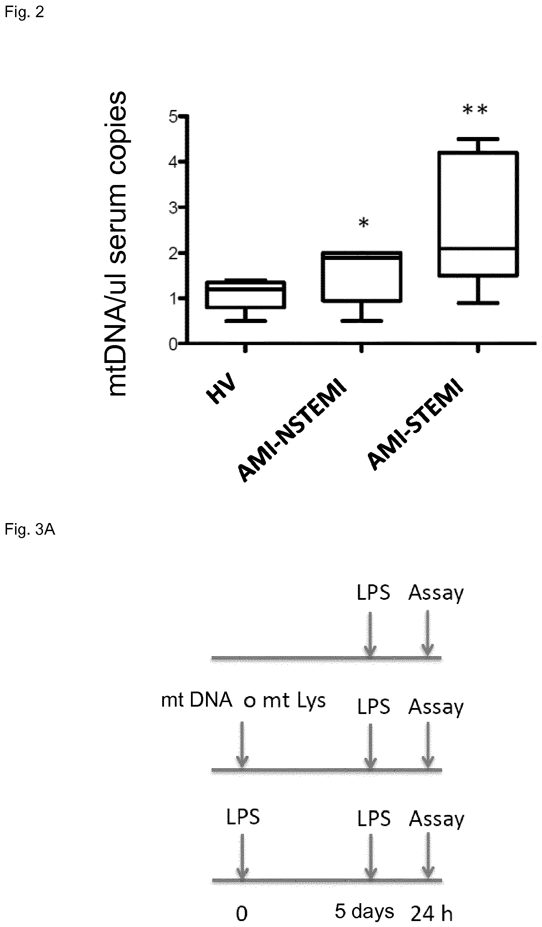

Fig. 2. Represents the levels of mitochondrial DNA in the serum of healthy volunteers (HV) and AMI patients. Values corresponding to HV, n=20; patients with: NSTEMI n= 24, STEMI n= 21. *p<0.05, **p<0.01 patients vs (ANOVA/Dunn) were represented.

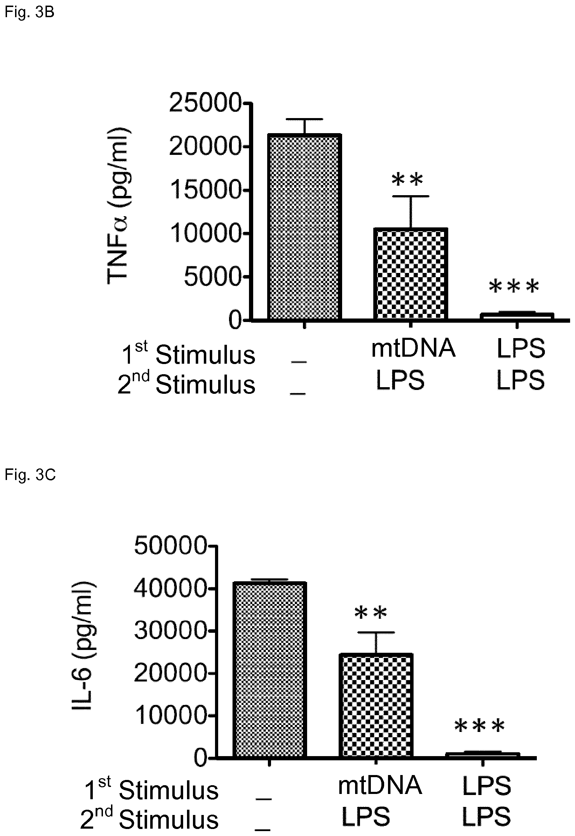

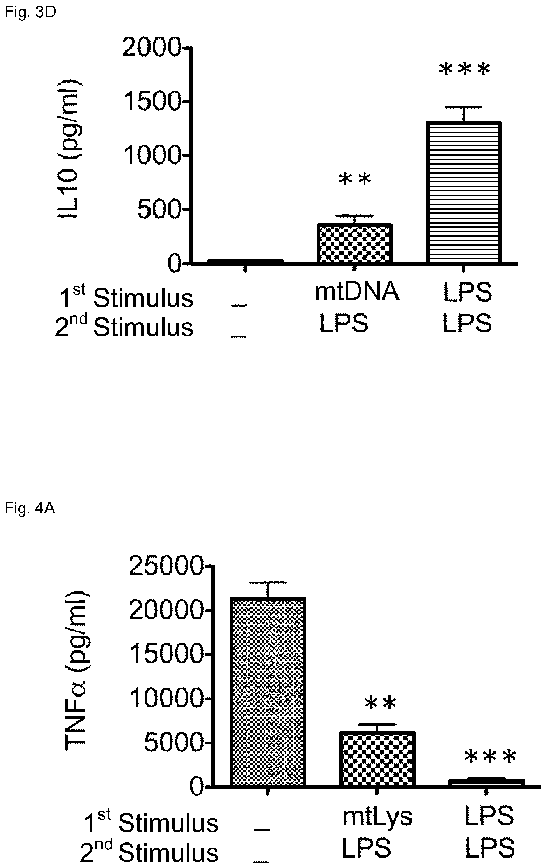

Fig. 3. Shows how exposure to mitochondrial DNA transforms monocytes into a phenotype M2 (tolerant/refractory). (A) Experimental design: Monocytes isolated from HV were exposed to mitochondrial DNA (mtDNA), LPS (10ng/ml) or received no treatment ("0", 1^{st} stimuli) for 5 days, then were stimulated again with LPS ("5 days", 2 ^{nd} stimuli). (B-D). At the assay moment (24 hours after LPS stimulus) cytokines TNF, IL6 and IL10 levels were measured in the supernatants of patient cell cultures vs. HV (measured by CBA or "Cytometric bead arrays", a flow cytometer technique where some microscopic beads attached to an antibody bind specifically to the desired cytokine).*p<0.05, **p<0.01, ***p<0.001.

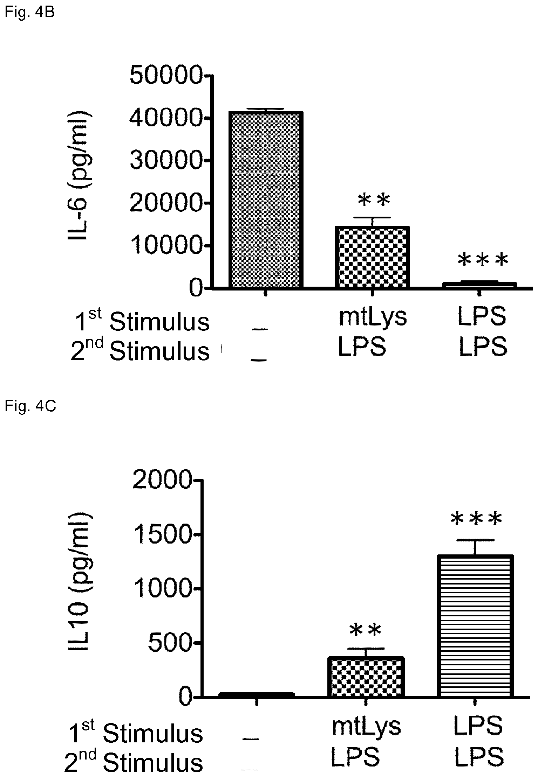

Fig. 4. Represents the modulation of human macrophages/monocytes due to their exposure to mitochondrial DAMPs (Damage Antigen Molecular Pattern), known as the mitochondrial lysate (mtLys). Macrophages/monocytes isolated from HV were exposed to mitochondrial DNA (mtDNA), LPS (10ng/ml) or were left without treatment for 5 days, then were returned to LPS stimulus, in accordance with the experimental outline of figure 3A. (A-C) Levels of cytokines TNF, IL6 and IL10 in the supernatant of cultures (measured by CBA).*p<0.05, **p<0.01, ***p<0.001 vs control (HV).

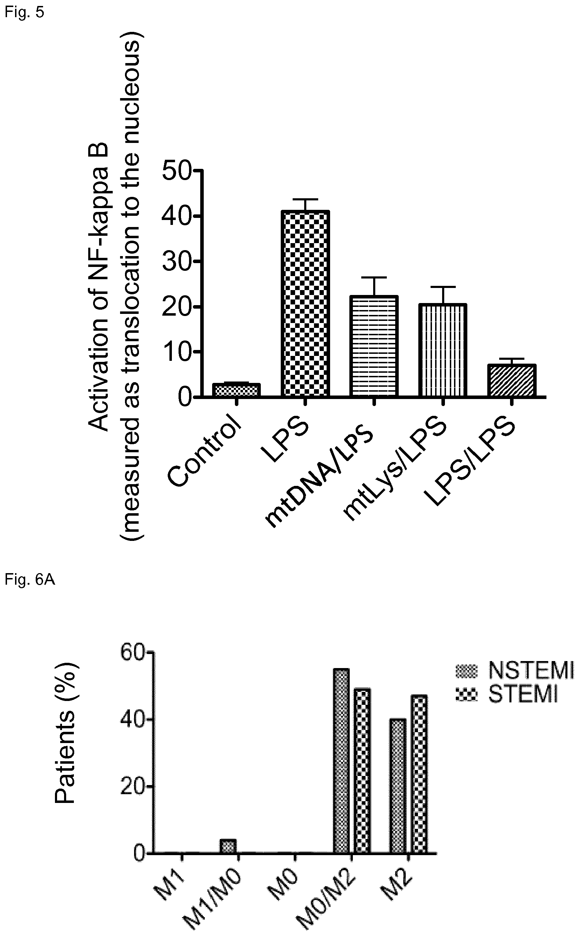

Fig. 5. Represents the modulation of NF-kappa-B activation on behalf of the mitochondrial DAMPs and mitochondrial DNA. Monocytes isolated from HV were exposed to mitochondrial DNA (mtDNA), mitochondrial DAMPs (mtLys), LPS or were left without treatment for 5 days, then were returned to LPS stimulus for 24 hours as explained in figure 3 ("control", monocytes with no treatment; "LPS" monocytes treated only after 5 days with LPS; "mtDNA/LPS", monocytes treated in a first stimulus with mtDNA and in a second stimulus with LPS; "mtLys/LPS" monocytes treated in a first stimulus with mtLys and in a second stimulus with LPS; "LPS/LPS", monocytes treated in a first stimulus with LPS and in a second stimulus with LPS).The quantification of the translocation of NF-kappa-B (p65) to the nucleus made by confocal microscopy is represented *p<0.05, **p<0.01 vs control (HV).

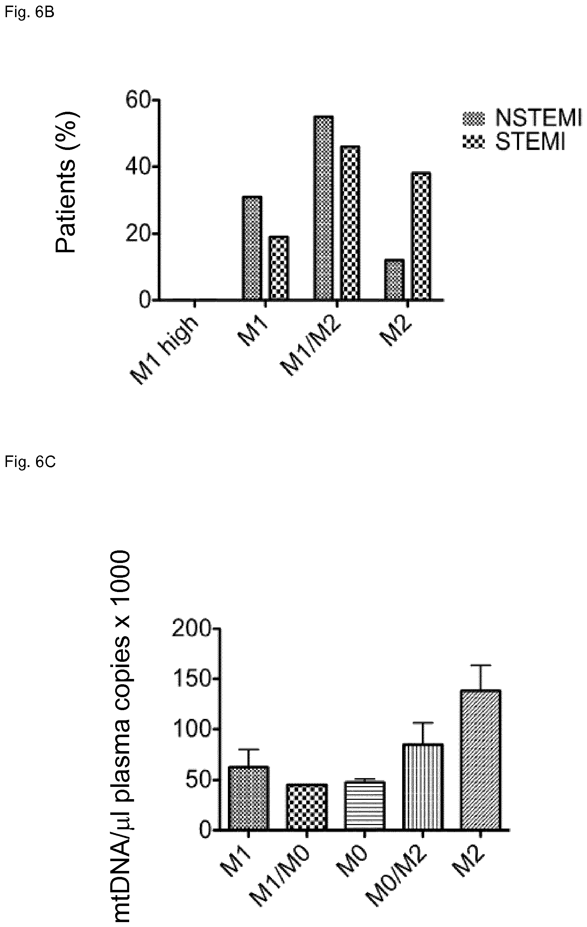

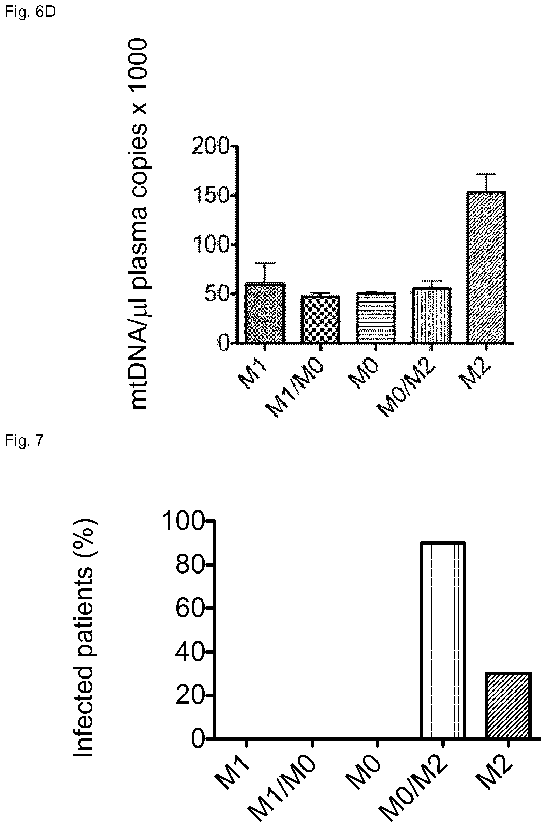

Fig. 6.Represents the score index (M1/M2) of the AMI patients (M1 is a classical phenotype, while M2 is a tolerant/refractory phenotype). Patients were classified in accordance with their M1/M2 index in the basal condition (A), after LPS stimulation (3h, 10ng/ml). (C-D) represent the number of mtDNA copies found per millimeter of blood and its correspondence with the M1/M2 score, of macrophages /monocytes.
Fig. 7. Represents the percentage (%) of patients, classified according to the score on figure 6, that got infected.

### EXAMPLES

Example 1: determining the risk of developing an infectious disease in Acute Myocardiac Infartion (AMI) patients.

The study comprised 45 consecutive AMI patients admitted to the Cardiology Service of *Hospital Universitario La Paz* where they underwent a coronary angiography. 21 out of the 45 patients (46.7%) had an elevated ST segment in their ECG (electrocardiogram) and 24 patients (53.3%) had no elevated ST segment. Twenty healthy volunteers (HV) of similar ages and gender and without a history of established Coronary Heart Disease (CHD) or cardiovascular risk factors were included as controls. A follow-up study of all the AMI patients that took part in this study was carried out. 7 out of 45 patients were admitted to hospital again with an infection during the three following months after their first admittance.

Over the three months after being released from hospital, six patients with AMI had an infection in their urinary tract (4 patients had STEMI, 2 patients had NSTEMI) and one patient in the group of STEMI caught community acquired pneumonia (that is to say, a non-nosocomial pneumonia).

Surprisingly patients who had elevated levels mitochondrial compounds in their blood, had a higher risk of developing an infectious disease.

This study excluded patients with acute infections, chronic inflammatory diseases, immunosuppression, active and /or chronic infectious diseases, a history of kidney disease, liver diseases or malignant diseases, in the past 3 months or under immunesuppressant therapy were also excluded.

Also, surprisingly, circulating macrophages and monocytes in these patients showed a refractory state of activation (or tolerance to infections) similar to that described in the so-called "tolerance to endotoxins" (TE).

Therefore, a previously established model of human tolerance to endotoxin (BiswasSK y López-Collazo E. 2009 Cell 30(10):475-487) was used to understand the reason why these macrophages and monocytes were activated.

It has been established that monocytes/macrophages can show different states of activation. They can polarize, according to micro-environmental signals, into two main groups: classically activated macrophages (M1), whose activating stimulus is typically IFN-γ, and alternatively activated macrophages (M2), activated by exposure to IL4, IL13 or IL10. M1 cells show strong antimicrobial properties, they promote the Th1 response and mainly produce pro-inflammatory cytokines, such as TNF-α, IL-1β and IL6. By contrast, M2 cells can be partially identified by the expression of the Scavenger receptor CD163, they produce cytokines and anti-inflammatory molecules, such as IL10, CCL18 and TGF-β, and cytokines, chemokines and supportive soluble factors associated to Th2 effector function. (Biswas SK y López-Collazo E. 2009 Cell 30 (10):475-487).

This M2 activation was found in the invention that surprisingly coincides with the phenomenon called "refractory state" or "tolerance to infections".

In order to study macrophages/monocytes functions sixty milliliters of blood samples were used. They were extracted from a peripheral vein of all the AMI patients, after their first 30 hours in hospital (average 36 ± 6) to avoid an initial acute pro-inflammatory response of ischemic injury.

The mitochondrial DNA from the patients' plasma samples was extracted in duplicate with the QlAamp DNA Mini Kit (QIAGEN, GmbH, Germany) according to the manufacturer's protocol. The "mtDNA 12S ribosomal RNA sonda Taq Man" (Applied Biosystems) (SEQ ID NO: 4) probe was used and primers (SEQ ID NO: 2 and SEQ ID NO: 3) of that same manufacturer were used. The average of the number of copies of mtDNA obtained in both extractions from the same sample of serum was calculated and the results were expressed as the number of copies of mtDNA/µl of serum (Figure 2).

The balance of M1/M2 was calculated by using the mRNA expression levels of TNFα and IL-10 from macrophages of patients with AMI in basal condition and after 3 h of stimulation with lipopolysaccharides (LPS) in culture (3 hours, 10ng/ml) (Figures 1A-F).

In baseline conditions, it was considered whether in M1 the TNFα levels were higher than the average ± SD of healthy volunteers (HV) and if the IL10 level values were below the average - SD of the HV's value. Quite the opposite, an M2 status was given to patients with TNF levels below the average - SD in high tension and the IL10 levels were higher than average + SD of HV. Those patients who had mRNA levels of both cytokines within the interval of average ± SD of HV were considered as M0. Since macrophages polarization is continuous, there are two intermediary states, M1/M0 (with high TNFα and IL10, as in a HV) and M0/M2 (with TNFα, as in a HV and high IL10).

After LPS exposure, no sample had its macrophages in M0 state, as it indeed happened in the case of the HV. Since any healthy subject who has had an infarct would have a classic M1 pro-inflammatory response, all patients that had mRNA levels of both cytokines within the interval of the average ± SD of HV after stimulation with LPS were considered as M1. All patients with a TNFα higher than the average + SD and IL10 below the average - SD were classified as "high" M1, due to its high TNFα level, in order to differentiate them from those with little increases that could be developed by chance. M2 and M2/M0 were calculated as described for the baseline condition.

Statistical analysis: the number of experiments analyzed is shown in each figure. Data were obtained from a minimum of three experiments and were expressed as average ± SD. The statistical significance was calculated with the one-way ANOVA analysis or the unpaired t-test, depending on the case. Spearman rank correlations were made for the non-normal data distribution. Differences were considered significant with p values <0,05 using *software Prism 5.0* (GraphPad, San Diego, CA, USA).

The angiographic, lab and clinical characteristics of patients under study were also analyzed. Quantitative coronary angiography revealed that 10 patients had lesions in one artery, 19 had lesions in two arteries, and 16 had lesions in three arteries. There was no significant difference regarding the degree of severe coronary atherosclerotic stenosis among the patients that had STEMI and NSTEMI. The average PCR values of IL-6 were significantly higher in AMI patients than in HV.

The anti-inflammatory response of circulating macrophages/monocytes correlated with the severity of AMI patients. In order to study the innate immune responses in AMI patients, firstly the mRNA's basal levels of pro-inflammatory factors (IL6 and TNFα), were quantified, as well as anti-inflammatory genes (IL10) expressed by circulating monocytes in patients and HV (Figure 1). Our data show that patients with more serious lesions and stenosis (STEMI and NSTEMI) showed lower levels of pro-inflammatory cytokines pertaining to M1 macrophage activators (Figure 1A-D). On the contrary, M2 anti-inflammatory factors, IL10 were higher in the macrophages of STEMI patients in comparison with HV (Figure 1E-F). These findings suggest that macrophages of STEMI patients had an M2 phenotype. This fact was verified when cells were stimulated *ex vivo* with LPS (Figure 3A). After 3 h of LPS stimulation, the macrophages of STEMI and NSTEMI patients were unable to increase their baseline expression of IL-6 or TNFα, as HV had done (Figure 3B-C). Nevertheless, only the cells of STEMI patients showed an increase of IL10 mRNA levels (Figure 2D).This data shows that macrophages of AMI (STEMI and NSTEMI) patients are circulating and have an anti-inflammatory phenotype that nearly coincides with the M2 activation previously described. One must take into account that the inflammatory profile of these patients was analyzed after the first 30 hours of hospitalization, an average time of 30± 6h.

Having established that the circulating macrophages of AMI patients were in a refractory state, similar to the more obvious state of tolerance to endotoxins found in STEMI patients (this protocol takes over 1 week to produce results), we proceeded to quantify the plasma levels of mtDNA in all our AMI patients. While mtDNA levels in plasma of NSTEMI patients were slightly higher to HV, those of STEMI patients were markedly higher (~ 3.5 times) in comparison to HV (Figure 2). To verify whether there was an association between the plasma levels of mitochondrial DNA and the M2 phenotype shown in STEMI patients, a correlation between the expression of cytokines and mtDNA plasma was analyzed. A significant correlation was found between mitochondrial DNA and TNF levels following LPS exposure (negative, Figure 2A) and the IL10 at baseline levels (positive, Figure 2C). This data confirms the existence of a marked increase of mtDNA in patients with severe coronary artery stenosis and this fact correlates with an M2 phenotype of its macrophages. The prolonged exposure of macrophages to mitochondrial DNA induces an M2 phenotype and the risk of a secondary infection as the patient is in a refractory state.

Therefore, plasma levels of mitochondrial DNA are higher in patients with coronary artery stenosis and there is an increase of multiple coronary lesions or STEMI patients as opposed to those who have no tissue lesions or NSTEMI (Figure 2).

To confirm these results, monocytes/macrophages of peripheral blood from HV were incubated for 5 days, with 5µg/ml of mtDNA isolated from the human cell line HeLa. Next, the cytokine production was measured in culture supernatants. The mtDNA induced a slight inflammatory response, which increased the pro-inflammatory cytokine production.

To verify if this soft response could induce an M2 polarization in the macrophage, after a pre-incubation time with mtDNA, cells from peripheral blood and specifically the macrophages of HV were stimulated with 10ng/ml LPS (experimental design in Figure 3A). This design was made to reproduce clinical conditions in which circulating monocytes/macrophages are in touch with mtDNA for a period of time longer than 30 hours. While the untreated cells produced big amounts of pro-inflammatory cytokines after LPS stimulation, pre-incubation with mtDNA led to a significant reduction in the production of this response (Figure 4A-B). This alteration of cytokine production was also found in LPS (instead of mtDNA) pre-stimulated macrophages, which we have used as a positive control of the M2 phenotype. By contrast, the M2 type anti-inflammatory protein IL10 increased upon pre-treating cells with mtDNA, as well as with a LPS pre-treatment (Figure 4C).These results show that, although mtDNA initially unchains pathways of inflammatory cytokines, it later, leads to an anti-inflammatory response.

Although we have proven the purity of the mitochondrial DNA extracts and that its contamination by nuclear DNA was less than 0.1% ( measured by quantitative PCR), we included nuclear DNA (nuDNA) as a stimulation control, of any genomic DNA waste present in the mitochondrial DNA extracts in which no stimulation was observed. Therefore, the results obtained from the mtDNA extracts, can be solely attributed to the mtDNA's action in the macrophage.

Overall, our results reveal that mt DNA tolerance is similar to the tolerance mediated by endotoxins. These results show the situation observed in AMI patients (more specifically 4 STEMI patients, 2 NSTEMI patients), that had high levels of circulating mitochondrial DNA (Figure 2) and exhibited tolerance to infections.

In baseline conditions, macrophages in all patients that later caught an infection were classified as having an M2 or intermediate M2 response (Figure 6A). After LPS stimulation, macrophages of all patients showed an intermediate M2 response (Figure 6BV). According to these results, mtDNA levels were higher in patients with an M2 phenotype as shown in Figures 6C-D.

Also the levels of inflammatory proteins were measured in the plasma collected in the first record, both from patients that later had an infection or HV (data not shown). Following the hierarchical grouping technique, all patients who had a later infection, with the exception of one, were grouped together and showed lower levels of inflammatory factors in their plasma that HV, thus reflecting a change in the anti-inflammatory response.

Therefore, we observed a high rate of later infection in patients with AMI and circulating monocytes/macrophages in stage M2.

Overall these results confirm that patients after an ischemic injury tend to go towards an anti-immune response of tolerance to infections or refractory state, and therefore are more likely to develop infections later on.

Example 2: Determining the risk of suffering infection in patients who have gone through major surgery.

In the same conditions as example 1, we studied a group of 75 patients. These patients were going to have major surgery. They had no immune suppression, infections, coronary diseases, no viral infections, had had no transplants, and had had no prior disease worth mentioning. They went through major surgery by medical prescription, like for example appendicitis, cardiovascular repairs, intestinal repairs, etc.

Blood was extracted from patients 5±2 hours prior surgery and 30±6 hours after surgery.

On one hand, circulating monocytes/macrophages from a peripheral blood sample were purified. *In vitro* immunological cultures and stimulations were made in such a way that results were obtained only 8 days after extraction of the sample, in order to thus determine the patients' immune condition.

On the other hand, mitochondrial DNA presence in the patient's blood plasma was quantified, through the previously described protocol, the sample was obtained at the same time as peripheral blood for the cell cultures. Mitochondrial DNA determination results were obtained within a few hours (3 to 5 hours).

Also the results of 20 healthy volunteers were obtained to be used as healthy controls. These samples were analyzed as previously described in example 1 to determine the tolerance to infections. Patients were monitored for three months after their operation.

A correlation between the mitochondrial DNA in the plasma of patients and the refractory state was observed. The patients who had blood mitochondrial DNA levels higher than 4•10⁴ copies of mtDNA/µl prior to the operation, developed post-operative infections (of gram-negative as well as gram-positive bacteria and even there was a case of fungi infection) in 72% of cases (13/18). These results were correlated with the tolerance to infections results obtained days after by the culture of immune systems' cells *in vitro.* The detected infections were gram-negative ones such as Escherichia *coli* as well as gram-positive bacteria such as *Staphylococcus aureus.* Also with the post-operative sample, we could predict whether the patient during his/her evolution was likely to develop an infection. If a patient after an operation remains in a refractory state special care and antibiotic treatment should be administered to avoid post-operative sepsis. In this case 28 patients were detected having high levels of circulating mitochondrial DNA after the operation, among these over 60% (17 patients) developed a post-operative infection.

Therefore, the present invention shows a correlation between the immunological refractory state of a patient before and after an operation, and the risk of developing an infection after an operation.

Example 3: Determining the risk of developing an infectious disease in cerebral infarction/ischemia patients.

In the same conditions as example 1, we studied a group of 75 patients. These patients had suffered cerebral ischemia or infarction within the 30 ± 6 hours previous to the extraction of the blood sample. They had no immune suppression, infections, coronary diseases, no viral infections, had had no transplants, and had had no prior disease worth mentioning. They had suffered a cerebrovascular disease or accident leading to bleeding or vascular obstruction caused by, for example, a vessel rupture, clot formation, etc.

Blood samples were extracted from patients 30 ± 6 hours after the cerebrovascular disease/accident.

On one hand, circulating monocytes/macrophages were purified in a peripheral blood sample. *In vitro* immunological cultures and stimulations were carried out in such a way that results were obtained only 8 days after extraction of the sample, in order to thus determine the patients' immune condition.

On the other hand, mitochondrial DNA presence in the patient's blood plasma was quantified, through the previously described protocol. The sample was obtained at the same time as peripheral blood for the cell cultures. Mitochondrial DNA determination results were obtained within a few hours (3 to 5 hours).

Also the results of 20 healthy volunteers were obtained to be used as healthy controls. These samples were analyzed as previously described in example 1 to determine the tolerance to infections. Patients were monitored for three months after their operation.

A correlation between the mitochondrial DNA in the plasma of patients and the refractory state was observed. The patients who had blood mitochondrial DNA levels higher than 4•10⁴ copies of mtDNA/µl after cerebrovascular disease/accident developed post-operative infections (of gram-negative as well as gram-positive bacteria and even there was a case of fungi infection) in 50% of cases (7/14). These results were correlated with the tolerance to infections results obtained days after by the *in vitro* culture of immune system's cells. The detected infections were gram-negative ones such as *Neiseria spp* as well as gram-positive bacteria such as *Streptococcus spp.*

Therefore, the present invention shows a correlation between the immunological refractory state of a patient after a cerebrovascular disease/accident and the risk of developing an infection after such accident or disease.

## Claims

1. *In vitro* method to determine the risk of developing an infectious disease before it has occurred comprising
(i) Quantifying the mitochondrial DNA in a biological sample of a subject, preferably a human being,
(ii) Comparing the value of the concentration of the mitochondrial DNA obtained in step (i) with the value of the concentration of the mitochondrial DNA from a control sample in order to find a significant difference between them, and
(iii) associating the significant difference with the risk of developing an infectious disease,
wherein
- a difference is considered to be significant when the value of the sample under observation is at least 2 times higher than the average +/- standard deviation of the control sample, and
- the subject does not suffer from an infection and has not received any immunosuppressed treatment nor has had a transplant.

2. Method according to claim 1, wherein the infectious disease is sepsis.

3. Method according to claims 1 or 2, wherein the subject has suffered an acute myocardial infarction.

4. Method according to any of claims 1 to 3, wherein the subject has suffered a cerebral infarction.

5. Method according to any of claims 1 to 4, wherein the subject will go or has gone through surgery.

6. Method according to any one of claims 1 to 5, wherein the mitochondrial DNA is gene 12S rRNA.

7. Method according to any of claims 1 to 6, wherein the biological sample is blood plasma, blood serum or blood.

8. Method according to any of claims 1 to 7, wherein the detection and/or quantification of the mitochondrial DNA is carried out by one of the techniques selected from the list consisting of PCR, chromatography, and spectroscopy.

9. Method according to claim 8, wherein the PCR used is a Quantitative PCR.

10. Method according to claim 9, wherein the Quantitative PCR is carried out by using primers comprising sequences SEQ ID NO: 2 and SEQ ID NO: 3 and a probe comprising sequence SEQ ID NO: 4.

11. Use of a kit that comprises primers comprising sequences SEQ ID NO: 2 and SEQ ID NO: 3 and a probe comprising sequence SEQ ID NO: 4, in order to predict the risk of developing an infectious disease in a subject who does not suffer from infection and has not received any immunosuppressed treatment nor has had a transplant.

12. Use according to claim 11, wherein the infectious disease is sepsis.

## Patentansprüche

1. *In vitro*-Verfahren zur Bestimmung des Risikos, eine infektiöse Krankheit zu entwickeln, bevor sie aufgetreten ist, umfassend
(i) Quantifizieren der mitochondrialen DNA in einer biologischen Probe eines Subjekts, vorzugsweise eines Menschen,
(ii) Vergleichen des Werts der Konzentration der in Schritt (i) erhaltenen mitochondrialen DNA mit dem Wert der Konzentration der mitochondrialen DNA aus einer Kontrollprobe, um einen signifikanten Unterschied zwischen ihnen zu finden, und
(iii) Assoziieren des signifikanten Unterschieds mit dem Risiko, eine infektiöse Krankheit zu entwickeln,
wobei
- ein Unterschied als signifikant angesehen wird, wenn der Wert der beobachteten Probe mindestens 2-mal höher ist als die durchschnittliche Plus/Minus-Standardabweichung der Kontrollprobe, und
- das Subjekt nicht an einer Infektion leidet und weder eine immunsupprimierte Behandlung noch eine Transplantation erhalten hat.

2. Verfahren nach Anspruch 1, wobei die infektiöse Krankheit Sepsis ist.

3. Verfahren nach Anspruch 1 oder 2, wobei das Subjekt einen akuten Myokardinfarkt erlitten hat.

4. Verfahren nach einem der Ansprüche 1 bis 3, wobei das Subjekt einen Hirninfarkt erlitten hat.

5. Verfahren nach einem der Ansprüche 1 bis 4, wobei das Subjekt einer Operation unterzogen wird oder unterzogen wurde.

6. Verfahren nach einem der Ansprüche 1 bis 5, wobei die mitochondriale DNA das Gen 12S rRNA ist.

7. Verfahren nach einem der Ansprüche 1 bis 6, wobei die biologische Probe Blutplasma, Blutserum oder Blut ist.

8. Verfahren nach einem der Ansprüche 1 bis 7, wobei der Nachweis und/oder die Quantifizierung der mitochondrialen DNA durch eine der Techniken durchgeführt wird, die aus der Liste ausgewählt sind, die aus PCR, Chromatographie und Spektroskopie besteht.

9. Verfahren nach Anspruch 8, wobei die verwendete PCR eine quantitative PCR ist.

10. Verfahren nach Anspruch 9, wobei die quantitative PCR unter Verwendung von Primern, die die Sequenzen SEQ ID NO: 2 und SEQ ID NO: 3 umfassen, und einer Sonde, umfassend die Sequenz SEQ ID NO: 4, durchgeführt wird.

11. Verwendung eines Kits, das Primer, die die Sequenzen SEQ ID NO: 2 und SEQ ID NO: 3 umfassen, und eine Sonde, umfassend die Sequenz SEQ ID NO: 4, umfasst, um das Risiko der Entwicklung einer infektiösen Krankheit bei einem Subjekt vorherzusagen, das nicht an einer Infektion leidet und weder eine immunsupprimierte Behandlung noch eine Transplantation erhalten hat.

12. Verwendung nach Anspruch 11, wobei die infektiöse Krankheit Sepsis ist.

## Revendications

1. Procédé *in vitro* pour déterminer le risque de développer une maladie infectieuse avant qu'elle ne se déclare comprenant
(i) la quantification de l'ADN mitochondrial dans un échantillon biologique d'un sujet, de préférence un être humain,
(ii) la comparaison de la valeur de la concentration de l'ADN mitochondrial obtenue à l'étape (i) avec la valeur de la concentration de l'ADN mitochondrial provenant d'un échantillon témoin afin de trouver une différence significative entre eux et
(iii) l'association de la différence significative au risque de développer une maladie infectieuse,
dans lequel
- une différence est considérée comme étant significative lorsque la valeur de l'échantillon observé est au moins 2 fois supérieure à la moyenne +/- l'écart-type de l'échantillon témoin et
- le sujet ne souffre pas d'une infection et n'a pas reçu de quelconque traitement immunosuppresseur ni eu une transplantation.

2. Procédé selon la revendication 1, dans lequel la maladie infectieuse est la septicémie.

3. Procédé selon les revendications 1 ou 2, dans lequel le sujet a été sujet à un infarctus du myocarde aigu.

4. Procédé selon l'une quelconque des revendications 1 à 3, dans lequel le sujet a été sujet à un infarctus cérébral.

5. Procédé selon l'une quelconque des revendications 1 à 4, dans lequel le sujet va subir ou a subi une intervention chirurgicale.

6. Procédé selon l'une quelconque des revendications 1 à 5, dans lequel l'ADN mitochondrial est l'ARNr du gène 12S.

7. Procédé selon l'une quelconque des revendications 1 à 6, dans lequel l'échantillon biologique est du plasma sanguin, du sérum sanguin ou du sang.

8. Procédé selon l'une quelconque des revendications 1 à 7, dans lequel la détection et/ou la quantification de l'ADN mitochondrial sont réalisées par l'une des techniques choisies dans la liste constituée de la PCR, la chromatographie et la spectroscopie.

9. Procédé selon la revendication 8, dans lequel la PCR utilisée est une PCR quantitative.

10. Procédé selon la revendication 9, dans lequel la PCR quantitative est réalisée à l'aide d'amorces comprenant les séquences SEQ ID N° 2 et SEQ ID N° : 3 et d'une sonde comprenant la séquence SEQ ID N° : 4.

11. Utilisation d'un kit qui comprend des amorces comprenant les séquences SEQ ID N° : 2 et SEQ ID N° : 3 et une sonde comprenant la séquence SEQ ID N° : 4, afin de prédire le risque de développer une maladie infectieuse chez un sujet qui ne souffre pas d'infection et n'a pas reçu de quelconque traitement immunosuppresseur ni eu une transplantation.

12. Utilisation selon la revendication 11, dans laquelle la maladie infectieuse est la septicémie.
